# EUROPEAN PATENT SPECIFICATION

(11) **EP 2 894 153 B1**
(45) Date of publication and mention of the grant of the patent: **04.10.2017**
(21) Application number: 13836084.7
(22) Date of filing: 14.08.2013
(51) Int. Cl.: C07D 251/62

(54) **MELAMINE PURIFICATION PROCESS**
VERFAHREN ZUR MELAMINREINIGUNG
PROCÉDÉ DE PURIFICATION DE MÉLAMINE

(30) Priority: 10.09.2012 RU 2012138777
(43) Date of publication of application: 15.07.2015
(73) Proprietor: Otkrytoe Aktsionernoe Obschestvo Research & Design Institute Of Urea And Organic Synthesis Products (OAO NIIK), Nizhny Novgorod Region 606008 (RU)
(72) Inventor: NIKOLAEV, Evgeny Yuryevich, Nizhny Novgorod Region Dzerzhinsk 606037 (RU); SKUDIN, Alexei Georgievich, Nizhny Novgorod Region Dzerzhinsk 606009 (RU); PECHNIKOVA, Galina Nikolaevna, Krasnaya Gorka Settlement Dubki Village Nizhny Novgorod Region 606070 (RU); PROKOPYEV, Aleksandr Alekseevich, Nizhny Novgorod Region Volodarsk 606070 (RU); KUZNETSOV, Nikolai Mikhailovich, Nizhny Novgorod Region Dzerzhinsk 606000 (RU); KOSTIN, Oleg Niknlaevich, g. Nizhny Novgorod 603010 (RU); ESIN, Igor Veniaminovich, Nizhny Novgorod Region Dzerzhinsk 606000 (RU)
(74) Representative: Vossius & Partner Patentanwälte Rechtsanwälte mbB
(86) International application number: PCT/RU2013/000703
(87) International publication number: WO 2014/038980

(56) References cited:
- EP-B1- 1 444 214
- WO-A1-01/36397
- RU-C2- 2 304 579
- RU-C2- 2 367 656
- SU-A1- 82 174
- US-A- 2 341 180
- US-A- 3 132 143
- US-A- 3 300 492
- US-A1- 2004 162 429

## Description

### Field of the Invention

The present invention relates to a method for purifying melamine obtained by pyrolysis of urea according to a high-pressure non-catalytic process. More particularly, the invention relates to a method for purifying melamine from condensation products formed during melamine synthesis and separation, by its treatment in aqueous solution.

### Background of the Invention

It is known that unpurified melamine obtained in the high pressure melamine synthesis contains condensation products such as melam and melem resulting from melamine deammonolysis reactions.

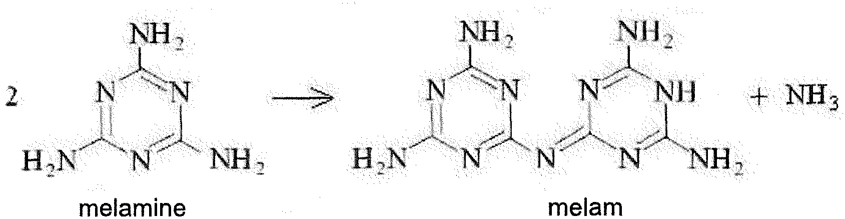

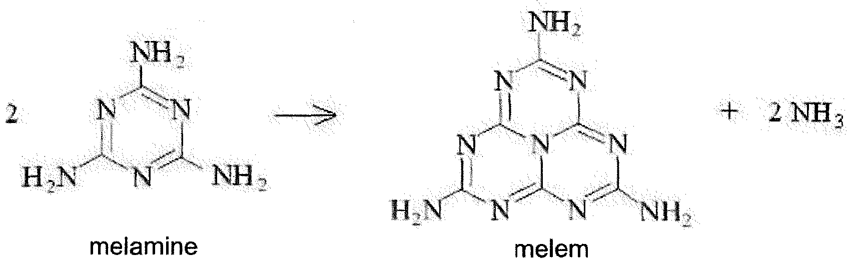

Melam and melem are highly undesirable impurities due to their negative impact on the quality of melamine-formaldehyde resins. In most non-catalytic melamine production processes these impurities are removed by hydrolysis in aqueous solutions and in presence of various reagents. As a result, melamine and hydrolysis products are formed. These products are soluble in alkaline or ammonia solutions allowing them to be easily separated from melamine.

The known method of melamine purification involves treating of an aqueous suspension of the unpurified melamine in presence of ammonia and carbon dioxide, which are formed in large quantities during melamine production (US 3132143, C07D 251/60, C07D 251/62, 1964).

In this patent, an aqueous suspension of melamine is formed when cooling (quenching) the product coming out of the high pressure reactor. The product contains melamine melt, ammonia and carbon dioxide, and the quenching is carried out with an aqueous solution containing ammonia and carbon dioxide.

In the Example 3 of the patent, melamine aqueous suspension containing ammonia and carbon dioxide is maintained at a temperature of 150°C for 60 minutes in the bottom part of the cooling unit, to achieve decomposition of condensation products.

A disadvantage of this method is the low degree of melamine purification: melamine product contains 0.2% of condensation products (named as insoluble impurities in Example) even after its recrystallization.

Various options of melam and melem hydrolysis in ammonia solutions are presented in patents. Thus, the known is a method of purifying melamine when subjecting unpurified melamine aqueous solution to a reaction with ammonia in an amount of 5-25% (preferably 12-15%). The reaction takes place at a temperature in a range of 130-250°C (preferably 160-180°C) and high pressure, the mixture is left to react for some minutes (US 6774234, C07D 251/60, C07D 251/62, 2004).

Unpurified melamine aqueous solution is formed when cooling (quenching) with water the product coming out of the high pressure reactor and containing melamine melt, ammonia and carbon dioxide, followed by carbon dioxide removal by steam stripping.

To decompose the condensation products, ammonia is added to the obtained melamine aqueous solution, the mixture is left to react in a separate unit.

Example 1 of the patent shows the process of treating melamine aqueous solution in presence of ammonia (13%) at a temperature of 172°C and a pressure of 2.5 MPa within 15 minutes. The condensation products content after treatment is less than 10 mg/l (UVspectroscopy analysis results).

The disadvantage of the known method of melamine purification is the need for an expensive high pressure vessel and large amounts of ammonia used. At further stages, ammonia should be separated from an aqueous solution. Additional stage for ammonia evaporation from aqueous solution makes the process more complicated and requires a lot of energy, thus increasing production costs.

From the technical point of view, the method that is the closest to the proposed method is a known method of melamine purification by treating melamine aqueous solution at a temperature of 100-150°C by means of 0.05-0.5% alkali within 5-60 minutes (EP 1444214, C07D 251/62, 2004).

In this patent, the melamine melt coming from the reactor is being separated from gases and cooled (quenched) with an alkali aqueous solution, specifically, sodium hydroxide or potassium hydroxide.

To proceed decomposition of condensation products, aqueous alkali-containing melamine solution is kept at the bottom part of the cooling unit within a certain time. Optimally, the process of decomposition runs at a temperature of 130°C within 20-40 minutes. As described in the EP 1444214 specification, using this method provides final residual concentration of melam less than 1000 ppm, concentration of melem - less than 50 ppm. After further recrystallization, a high degree of purity of commercial melamine is obtained.

However, despite a relatively low temperature of condensation products decomposition (preferably 130°C), melamine losses can not be avoided by reason of its alkaline hydrolysis (May N., Onyon P.F., Chemistry and Industry, 1962, v.23, pp. 1147-1148).

### Summary of the Invention

The technical problem to be solved by the invention is improving melamine purification process, providing high degree of melamine purification from condensation products at minimum melamine loss.

The technical result that can be obtained when using the invention is reducing melamine decomposition degree at a high degree of decomposition of condensation products.

To achieve the technical result, there is provided a method for melamine purification including treatment of unpurified aqueous melamine solution at elevated temperature within 5-60 minutes, wherein the treatment is carried out at a temperature of 110-170°C in presence of alkali metal carbonate.

Sodium carbonate, potassium carbonate or a mixture thereof are used as alkali metal carbonate. As a source of alkali metal carbonate purified wastewater of melamine plant comprising alkali metal carbonate can be used. The optimum concentration of alkali metal carbonate in the unpurified melamine solution is 0.1-1.0%.

The prior art does not disclose hydrolysis process for condensation products in presence of alkali metal carbonate. An unexpected effect of high degree decomposition of condensation products with a minimum degree of decomposition of melamine is achieved.

### Description of the Preferred Embodiments

The invention is illustrated by the following examples of implementing proposed method of melamine purification.

### Example 1.

An aqueous solution containing 5% of melamine, 0.06% of melam, 0.0035% of melem and 0.3% of sodium carbonate is prepared using lab mixer. The concentration of melam and melem corresponds approximately to impurities content in the unpurified melamine melt, which composition is given in Example 1 of EP 1444214. The resulting solution is kept in an autoclave for 45 minutes at 145°C. After the solution is cooled down to room temperature, the degree of hydrolysis of melam, melem and melamine is determined by high performance liquid chromatography (HPLC). The degree of decomposition of melam is 96.5%, melem - about 100%, melamine - 1.5%. Ammeline, ammelide, cyamelluric acid and cyamelluric acid amides are identified as water-soluble sodium salts in hydrolysis products. These salts can be easily separated from melamine during crystallization.

### Example 2 (comparative)

0.3% of sodium hydroxide instead of sodium carbonate is added to melamine solution of Example 1. The solution is kept in an autoclave for 30 minutes at 130°C. The resulting solution is cooled to room temperature and analyzed by HPLC. The degree of decomposition of melam is 94.7%, melem - about 100%, melamine - 2.4%.

Thus, for the same degree of hydrolysis of condensation products presented in Example 1 and Example 2, the loss of melamine in Example 1 is greatly reduced and makes approximately 60% of melamine loss according to prior art.

### Example 3.

An aqueous solution containing 5% of melamine, 0.06% of melam, 0.035% of melem and 1.0% of sodium carbonate is prepared using lab mixer. The solution is kept in an autoclave for 60 minutes at 110°C. After further cooling to room temperature, the solution is analyzed by HPLC. The degree of decomposition of melam is 91.7%, melem - 98.6%, melamine - 1.1%.

### Example 4.

An aqueous solution containing 5% of melamine, 0.06% of melam, 0.0035% of melem and 0.1% of sodium carbonate is prepared using lab mixer. The solution is kept in an autoclave for 5 minutes at 170°C. Then the solution is cooled to room temperature and hydrolysis degree of melam, melem and melamine is determined by HPLC. The degree of decomposition of melam is 94.7%, melem - nearly 100%, melamine - 1.3%.

### Example 5.

An aqueous solution containing 5% of melamine, 0.06% of melam, 0.0035% of melem and 0.4% of sodium carbonate is prepared using lab mixer. The solution was kept in an autoclave for 30 minutes at 140°C. After further cooling to room temperature, melam, melem and melamine hydrolysis degree is determined by HPLC. The degree of decomposition of melam is 93.8%, melem - 99.2%, melamine - 1.4%.

It can thus be seen from the Examples that the method of the present invention provides high degree of purification from melam and melem at insignificant degree of hydrolysis of melamine.

### Industrial Applicability

The invention maybe used in the industrial melamine production process.

## Claims

1. A process for the purification of melamine by treatment of unpurified melamine aqueous solution at elevated temperature within 5-60 minutes **characterized in that** the treatment is being performed at a temperature of 110-170°C in presence of alkali metal carbonate.

2. The process according to claim 1 **characterized in that** alkali metal carbonate concentration in the unpurified melamine solution is 0.1-1.0%.

3. The process according to claim 1 **characterized in that** sodium carbonate and/or potassium carbonate is used as alkali metal carbonate.

4. The process according to claim 1 **characterized in that** purified wastewater of melamine production plant comprising alkali metal carbonate is used as a source of alkali metal carbonate.

## Patentansprüche

1. Verfahren zur Melaminherstellung durch Behandlung einer wässrigen Lösung von Rohmelamin bei erhöhter Temperatur 5-60 Minuten lang, **dadurch gekennzeichnet, dass** die Behandlung bei einer Temperatur von 110-170°C in Gegenwart von Alkalimetallkarbonat durchgeführt wird.

2. Verfahren gemäß Anspruch 1, **dadurch gekennzeichnet, dass** die Konzentration des Alkalimetallkarbonats in der Lösung des Rohmelamins 0,1-1,0% ist.

3. Verfahren gemäß Anspruch 1, **dadurch gekennzeichnet, dass** Natriumkarbonat und/oder Kaliumkarbonat als Alkalimetallkarbonat verwendet wird.

4. Verfahren gemäß Anspruch 1, **dadurch gekennzeichnet, dass** behandeltes Abwasser einer Melaminherstellungsanlage, das Alkalimetallkarbonat umfasst, als eine Quelle des Alkalimetallkarbonats verwendet wird.

## Revendications

1. Le procédé de purification de la mélamine qui prévoit un traitement de la solution aqueuse de mélamine non purifiée à une température élevée pendant 5-60 minutes **se caractérisant par** le traitement qui est effectué sous la température de 110-170°C en présence du carbonate de métal alcalin.

2. Le procédé conformément au paragraphe 1 se différant par la concentration du carbonate de métal alcalin de 0,1-1,0% dans la solution de mélamine non purifiée.

3. Le procédé conformément au paragraphe 1 se différant par le carbonate de sodium et/ou le carbonate de potassium qui est utilisé en qualité de carbonate de métal alcalin.

4. Le procédé conformément au paragraphe 1 se différant par l'eau usée recyclée de la production de mélamine contenant du carbonate de métal alcalin qui est utilisée en qualité d'une source de carbonate de métal alcalin.
